# EUROPÄISCHE PATENTSCHRIFT

(11) **EP 0 564 972 B1**
(45) Veröffentlichungstag und Bekanntmachung des Hinweises auf die Patenterteilung: **11.08.1999**
(21) Anmeldenummer: 93105336.7
(22) Anmeldetag: 31.03.1993
(51) Int. Cl.: C07K 7/18, A61K 38/08

(54) **Peptide mit Modifikationen am N-Terminus mit bradykininantagonistischer Wirkung**
Peptides with N-terminal modifications having bradykinin antagonistic activity
Peptides présentant des modifications N-terminales ayant une activité antagoniste pour la bradykinine

(30) Priorität: 04.04.1992 DE 4211406
(43) Veröffentlichungstag der Anmeldung: 13.10.1993
(73) Patentinhaber: HOECHST AKTIENGESELLSCHAFT, 65926 Frankfurt am Main (DE)
(72) Erfinder: Breipohl, Gerhard, Dr., W-6000 Frankfurt am Main 71 (DE); Henke, Stephan, Dr., W-6238 Hofheim am Taunus (DE); Knolle, Jochen, Dr., W-6239 Kriftel/Ts. (DE); Schölkens, Bernward, Prof. Dr., W-6233 Kelkheim/Ts. (DE); Alpermann, Hans-Georg, Dr., W-6240 Königstein/Ts. (DE); Gerhards, Hermann, Dr., W-6238 Hofheim/Ts. (DE); Wirth, Klaus, Dr., W-6239 Kriftel/Ts. (DE)

(56) Entgegenhaltungen:
- EP-A- 0 370 453
- EP-A- 0 413 277
- EP-A- 0 455 133
- WO-A-89/01781
- WO-A-91/09055
- J.M. Stewart and R.J. Vavrek, 'Bradykinin Chemistry: Agonists and Antagonists', in Proceedings of the International Conference Kinin 81, pages 585-589

## Beschreibung

Die Erfindung betrifft neue Peptide mit bradykinin-antagonistischer Wirkung sowie ein Verfahren zu deren Herstellung.

Bradykinin-antagonistische Peptide werden unter anderem in WO 86/07263 und den europäischen Patentanmeldungen Nr. 370 453, Nr. 413 277, Nr. 455 133 und Nr. 472 220 beschrieben.

Die vorliegende Erfindung beschreibt neue Peptide mit bradykinin-antagonistischer Wirkung der Formel I

Z - P - A - B - C - E - F - K - (D)Q - G - M - F' - I (I),

in welcher
- Z: Fmoc, Dibenzylacetyl, Cyclohexylcarbonyl, N,N-Dibenzyl-glycyl, 2-(4-Isobutylphenyl)propionyl, 2-R-(tert.-Butylsulfonylmethyl)-3-(1-naphthyl)propionyl, Indol-3-yl-acetyl, 4-Benzoyl-benzoyl, 1,8-Naphthalimidoacetyl, 7-Theophyllinacetyl oder N-Benzoyl bedeutet;
- P: für eine direkte Bindung, Aoc, ε-Aminohexanoyl, D-Aoc, Aeg(Fmoc), 4-Aminocyclohexylcarbonyl, 4-Aminomethylcyclohexylcarbonyl oder Oic steht;
- A: (D)- oder (L)-Arg, (D)- oder (L)-Lys oder eine Bindung bedeutet;
- B: Arg bedeutet;
- C: Pro-Hyp-Gly bedeutet;
- E: Thia bedeutet;
- F: Ser bedeutet;
- K: für eine direkte Bindung steht;
- Q: Tic bedeutet;
- M: für eine direkte Bindung steht;
- G: für cis-endo-, cis-exo-, trans-Octahydroindol-2-carbonsäure steht;
- F': Arg bedeutet und
- I: für OH steht;
sowie deren physiologisch verträgliche Salze.

Falls nicht anders angegeben, steht die Abkürzung eines Aminosäurerestes ohne einen Stereodeskriptor für den Rest in der L-Form (vgl. Schröder, Lübke, Peptides, Band 1, New York 1965, Seiten XXII-XXIII; Houben-Weyl, Methoden der Organischen Chemie, Band XV/1 und 2, Stuttgart 1974), wie z. B.
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, hArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val etc.

Als Salze kommen insbesondere Alkali- oder Erdalkalisalze, Salze mit physiologisch verträglichen Aminen und Salze mit anorganischen oder organischen Säuren wie z.B. HCI, HBr, H₂SO₄, H₃PO₄, Maleinsäure, Fumarsäure, Citronensäure, Weinsäure, Essigsäure in Frage.

Die Erfindung betrifft weiterhin ein Verfahren zur Herstellung von Peptiden der Formel I, das dadurch gekennzeichnet ist, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut,
in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihr physiologisch verträgliches Salz überführt.

Die Peptide der vorliegenden Erfindung wurden nach allgemein bekannten Methoden der Peptidchemie, siehe z.B. Houben-Weyl, Methoden der organischen Chemie, Band 15/2, bevorzugt mittels Festphasensynthese wie z.B. von B. Merrifield, J. Am. Chem. Soc. 85, 2149 (1963) oder R.C. Sheppard, Int. J. Peptide Protein Res. 21, 118 (1983) beschrieben oder durch äquivalente bekannte Methoden hergestellt. Als temporäre Aminoschutzgruppe werden Urethanschutzgruppen wie z.B. die tert.-Butyloxycarbonyl[Boc]- oder Fluorenylmethyloxycarbonyl[Fmoc]-Schutzgruppe verwendet.
Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis") zusätzlich geschützt, wobei in erster Linie Arg(Tos), Arg(Mts), Arg(Mtr), Arg(Pmc), Asp(OBzl), Asp(OBut), Cys(4-MeBzl), Cys(Acm), Cys(SBut), Glu(OBzl), Glu(OBut), His(Tos), His(Fmoc), His(Dnp), His(Trt), Lys(Cl-Z), Lys(Boc), Met(O), Ser(Bzl), Ser(But), Thr(Bzl), Thr(But), Trp(Mts), Trp(CHO), Trp(Boc), Tyr(Br-Z), Tyr(Bzl) oder Tyr(But) eingesetzt werden.

Die Festphasensynthese beginnt am C-terminalen Ende des Peptids mit der Kupplung einer geschützten Aminosäure an ein entsprechendes Harz. Derartige Ausgangsmaterialien können durch Verknüpfung einer geschützten Aminosäure mit einem mit einer Chlormethyl-, Hydroxymethyl-, Benzhydrylamino (BHA)-, Methylbenzhydrylamino (MBHA) - Gruppe modifiziertem Polystyrol- oder Polyacrylamid-Harz über eine Ester- bzw. Amidbindung erhalten werden. Die als Trägermaterial verwendeten Harze sind kommerziell erhältlich. BHA- und MBHA-Harze werden für gewöhnlich verwendet, wenn das synthetisierte Peptid am C-Terminus eine freie Carbamoylgruppe enthalten soll. Falls das Peptid eine sekundäre Amidgruppe am C-terminalen Ende enthalten soll wird ein Chlormethyl- bzw. Hydroxymethyl-Harz verwendet und die Abspaltung mit den entsprechenden Aminen durchgeführt. Will man z.B. das Ethylamid erhalten, kann das Peptid mit Ethylamin vom Harz abgespalten werden, wobei die Abspaltung der Seitenketten-Schutzgruppen nachfolgend durch andere geeignete Reagenzien erfolgt. Sollen im Peptid die tert.-Butyl-Schutzgruppen der Aminosäure-Seitenkette erhalten bleiben, so wird die Synthese mit der Fmoc-Schutzgruppe zur temporären Blockierung der zur Kettenverlängerung verwendeten Amino-Gruppe der Aminosäure unter Verwendung der z.B. bei R.C. Sheppard, J. Chem. Soc., Chem. Comm. 1982, 587 beschriebenen Methodik durchgeführt, wobei die Guanidino-Funktion des Arginins durch Protonierung mit Pyridinium-Perchlorat geschützt wird und der Schutz der anderen in der Seitenkette funktionalisierten Aminosäuren mit durch katalytische Transferhydrierung (A. Felix et al., J. Org. Chem. 13, 4194 (1978)) oder durch Natrium in flüssigem Ammoniak (W. Roberts, J. Am. Chem. Soc. 76, 6203 (1954)) abspaltbaren Benzylschutzgruppen erfolgt.

Nach Abspaltung der Aminoschutzgruppe der an das Harz gekuppelten Aminosäure mit einem geeigneten Reagenz, wie z.B. Trifluoressigsäure in Methylenchlorid im Falle der Boc-Schutzgruppe oder einer 20 %igen Lösung von Piperidin in Dimethylformamid im Falle der Fmoc- Schutzgruppe, werden die nachfolgenden geschützten Aminosäuren nacheinander in der gewünschten Reihenfolge aufgekuppelt. Die intermediär entstehenden N-terminal geschützten Peptidharze werden vor der Verknüpfung mit dem nachfolgenden Aminosäurederivat durch die vorbeschriebenen Reagenzien entblockiert.

Als Kupplungsreagenz können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid oder Uronium-Verbindungen (z.B. TBTU, TOTU) wie sie z.B. bei R. Knorr et al. in Tetrahedron Letters, Vol. 30, No.15, pp 1927-1930, 1989 oder in der europäischen Patentanmeldung Nr. 460 446 beschrieben sind. Die Kupplung kann dabei direkt durch Addition von Aminosäurederivat mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R. Geiger, Chem. Ber. 103, 2054 (1970)) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Aminosäurederivate mit einem der oben genannten Aktivierungsreagenzien kann in Dimethylformamid, N-Methylpyrrolidon oder Methylenchlorid oder einer Mischung aus den genannten Lösungsmitteln durchgeführt werden. Das aktivierte Aminosäurederivat wird üblicherweise in einem 1,5 bis 4 fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockierung der Aminogruppe des Peptidharzes durchzuführen.

Der erfolgreiche Ablauf der Kupplungsreaktion kann mittels der Ninhydrin-Reaktion, wie z.B. von E. Kaiser et al., Anal. Biochem. 34, 595 bei R.C. Sheppard, J. Chem. Soc., Chem. Comm. 1982, 587 beschrieben. Sollen einzelne tert.-Butyl bzw. Benzylseitenketten-Schutzgruppen erhalten bleiben, so verwendet man eine geeignete Kombination der Synthese- und Abspaltmethoden.

Für die Synthese von Peptiden mit einer C-terminalen Carbamoylgruppierung oder einer ω-Amino- bzw. ω-Guanidinoalkylgruppierung wird ebenfalls das von Sheppard beschriebene modifizierte Trägerharz verwendet. Nach der Synthese wird das in der Seitenkette vollgeschützte Peptid vom Harz abgespalten und anschließend in klassischer Lösungssynthese mit dem entsprechenden Amin bzw. ω-Aminoalkylamin oder ω-Guanidinoalkylamin umgesetzt, wobei gegebenenfalls vorhandene weitere funktionelle Gruppen in bekannter Weise temporär geschützt werden können.

Ein weiteres Verfahren zur Herstellung von Peptiden mit einer ω-Aminoalkylgruppierung ist in EP-A 264 802 beschrieben.

Die Peptide der vorliegenden Erfindung wurden vorzugsweise unter Verwendung der Festphasentechnik nach zwei generellen Schutzgruppentaktiken synthetisiert:

Die Synthese erfolgte mit einem automatischen Peptidsynthesizer Modell 430 A der Fa. Applied Biosystems unter Verwendung von Boc- bzw. Fmoc-Schutzgruppen zur temporären Blockierung der Aminogruppe.

Bei Verwendung der Boc-Schutzgruppe wurden für die Synthese die vom Hersteller des Gerätes vorprogrammierten Synthesezyklen benutzt.

Die Synthese der Peptide mit einer freien Carboxylgruppe am C-terminalen Ende erfolgte an einem mit der entsprechenden Boc-Aminosäure funktionalisiertem 4-(Hydroxymethyl)phenyl-acetamidomethylpolystyrolharz (R.B. Merrifield, J. Org. Chem. 43, 2845 (1978)) der Fa. Applied Biosystems. Für die Herstellung der Peptidamide wurde ein MBHA-Harz derselben Firma verwendet. Als Aktivierungsreagenzien dienten N,N'-Dicyclohexylcarbodiimid oder N,N'-Diisopropylcarbodiimid. Die Aktivierung erfolgte als symmetrisches Anhydrid, als HOBt-Ester oder HOObt-Ester in CH₂Cl₂, CH₂Cl₂ - DMF-Gemischen oder NMP. Für die Kupplung wurden 2-4 Äquivalente an aktiviertem Aminosäurederivat eingesetzt. Für Fälle in denen die Kupplung unvollständig verlief, wurde die Reaktion wiederholt.

Bei der Verwendung der Fmoc-Schutzgruppe zum temporären Schutz der Aminogruppe wurden für die Synthese mit dem automatischen Peptid-Synthesizer Modell 430A der Fa. Applied Biosystems eigene Syntheseprogramme eingegeben. Die Synthese erfolgte an einem p-Benzyloxybenzylalkohol-Harz (S. Wang, J. Am. Chem. Soc. 95, 1328 (1973)) der Fa. Bachem, das nach bekannter Methode (E. Atherton et al., J.C.S.Chem. Comm. 1981, 336) mit der entsprechenden Aminosäure verestert war. Die Aktivierung der Aminosäurederivate als HOBt- oder HOObt-Ester erfolgte direkt in den vom Gerätehersteller gelieferten Aminosäurecartridges durch Zugabe einer Lösung von Diisopropylcarbodiimid in DMF zu der vorher eingewogenen Mischung aus Aminosäurederivat und HOBt oder HOObt. Ebenfalls eingesetzt werden können in Substanz hergestellte Fmoc-Aminosäure-OObt Ester wie sie in EP-A 247 573 beschrieben sind. Die Abspaltung der Fmoc-Schutzgruppe erfolgte mit einer 20 %igen Lösung von Piperidin in DMF im Reaktionsgefäß. Der verwendete Überschuß an reaktivem Aminosäurederivat betrug 1,5 bis 2,5 Äquivalente. Falls die Kupplung nicht vollständig war, wurde sie wie bei der Boc-Methode wiederholt.

Die erfindungsgemäßen Peptide haben einzeln oder in Kombination eine bradykinin-antagonistische Wirkung, die in verschiedenen Modellen getestet werden kann (s. Handbook of Exp. Pharmacol. Vol. 25, Springer Verlag, 1970, S. 53-55), so z.B. am isolierten Rattenuterus, am Meerschweinschenileum oder an der isolierten Pulmonalarterie des Meerschweinchens.

Für die Testung der erfindungsgemäßen Peptide an der isolierten Arteria pulmonalis werden Meerschweinchen (Dunkin Hartley) mit einem Gewicht von 400 - 450 g durch Genickschlag getötet. Der Brustkorb wird geöffnet und die Arteria pulmonalis vorsichtig herauspräpariert. Das umliegende Gewebe wird sorgfältig entfernt und die Arteria pulmonalis in einem Winkel von 45° spiralig aufgeschnitten. Der Gefäßstreifen von 2,5 cm Länge und 3 - 4 mm Breite wird in einem 10 ml fassenden Organbad, das mit Ringerlösung gefüllt ist, fixiert.

| Zusammensetzung der Lösung in mmol/l | |
|---|---|
| NaCl | 154 |
| KCl | 5,6 |
| CaCl₂ | 1,9 |
| NaHCO₃ | 2,4 |
| Glukose | 5,0 |

Die Lösung wird mit 95 % O₂ und 5 % CO₂ durchperlt und auf 37 °C erwärmt. Der pH beträgt 7,4, die Vorlast am Gefäßstreifen beträgt 1,0 g.

Die isometrische Kontraktionsänderungen werden mit einem Hebelvorsatz und einem HF-Modem (Wegmesser) von Hugo Sachs erfaßt und auf einen Kompensationsschreiber (BEC, Goerz Metrawatt SE 460) registriert.

Nach 1 Stunde Äquilibrierung wird mit dem Versuch begonnen. Nachdem die Gefäßstreifen ihre maximale Empfindlichkeit gegenüber 2 x 10⁻⁷ mol/l Bradykinin erreicht haben - Bradykinin führt zu einer Kontraktion der Gefäßstreifen - werden die Peptide in den Dosen 5 x 10⁻⁸ - 1 x 10⁻⁵ mol/l jeweils 10 Minuten einwirken lassen und nach erneuter Gabe von Bradykinin die Abnahme des Effektes von Bradykinin gegenüber der Kontrolle verglichen.

Zur Erkennung eines partialagonistischen Effektes werden die Peptide in den Dosen 1 x 10⁻⁵ - 1 x 10⁻³ mol/l verwendet.

Die aus den Dosiswirkungskurven errechneten IC₅₀-Werte der erfindungsgemäßen Peptide sind in der Tabelle 1 aufgeführt.

**Tabelle 1**

| Bsp. Nr. | Sequenz | IC₅₀-Wert (Pulmonar -Arterie) |
|---|---|---|
| 1 | Fmoc-D-Arg-Arg-Pro-Hyp-Gly-Thia-Ser-D-Tic-Oic-Arg-OH | 9,9 x 10⁻⁹ |
| 2 | Fmoc-Aoc-D-Arg-Arg-Pro-Hyp-Gly-Thia-Ser-D-Tic-Oic-Arg -OH | 8,5 x 10⁻⁹ |
| 3 | Dibenzylacetyl-D-Arg-Arg-Pro-Hyp-Gly-Thia-Ser-D-Tic-Oic-Arg-OH | 1,7 x 10⁻⁸ |
| 4 | Cyclohexylcarbonyl-D-Arg-Arg-Pro-Hyp-Gly-Thia-Ser-D-Ti c-Oic- Arg-OH | 9,1 x 10⁻⁹ |
| 5 | Fmoc-ε-Aminocaproyl-D-Arg-Arg-Pro-Hyp-Gly-Thia-Ser-D-Tic-Oic-Arg-OH | 4,1 x 10⁻⁹ |
| 6 | N,N-Dibenzyl-Gly-D-Arg-Arg-Pro-Hyp-Gly-Thia-Ser-D-Tic-Oic-Arg-OH | 2,2 x 10⁻⁸ |
| 7 | Fmoc-D-Aoc-Arg-Arg-Pro-Hyp-Gly-Thi-Ser-D-Tic-Oic-Arg-OH | 2,2 X 10⁻⁸ |
| 8 | 2-(4-Isobutylphenyl)propionyl-Arg-Arg-Pro-Hyp-Gly-Thi-Ser-D-Tic-Oic-Arg-OH | 2,5 x 10⁻⁸ |
| 9 | (2-R-(tert.-butylsulfonylmethyl)-3-(1-naphthyl)propionyl-Arg-Arg-Pro-Hyp-Gly-Thi-Ser-D-Tic-Oic-Arg-OH | 1,2 x 10⁻⁸ |
| 10 | Indol-3-yl-acetyl-Arg-Arg-Pro-Hyp-Gly-Thi-Ser-D-Tic-Oic-Arg-OH | 8,8 x 10⁻⁹ |
| 11 | 2-(4-isobutylphenyl)propionyl-D-Arg-Arg-Pro-Hyp-Gly-Thi-Ser-D-Tic-Oic-Arg-OH | 1,8 x 10⁻⁸ |
| 12 | 2-(4-Isobutylphenyl)propionyl-6-aminohexanoyl-D-Arg-Arg-Pro-Hyp-Gly-Thi-Ser-D-Tic-Oic-Arg-OH | 7,8 x 10⁻⁹ |
| 13 | 6-(4-Benzoyl-benzoylamino)hexanoyl-D-Arg-Arg-Pro-Hyp-Gly-Thi-Ser-D-Tic-Oic-Arg-OH | 2,7 x 10⁻⁸ |
| 14 | Fmoc-Aeg(Fmoc)-D-Arg-Arg-Pro-Hyp-Gly-Thi-Ser-D-Tic-Oic-Arg-OH | 1,5 x 10⁻⁸ |
| 15 | Fmoc-(4-aminocyclohexylcarbonyl)-D-Arg-Arg-Pro-Hyp-Gly-Thi-Ser-D-Tic-Oic-Arg-OH | 2,5 x 10⁻⁸ |
| 16 | 1,8-Naphthalimidoacetyl-D-Arg-Arg-Pro-Hyp-Gly-Thi-Ser-D-Tic-Oic-Arg-OH | 7,8 x 10⁻⁹ |
| 17 | (2-R-(tert.-butylsulfonylmethyl)-3-(1-naphthyl)-propionyl-D-Arg-Arg-Pro-Hyp-Gly-Thi-Ser-D-Tic-Oic-Arg-OH | 8,3 x 10⁻⁹ |
| 18 | Indol-3-yl-acetyl-D-Arg-Arg-Pro-Hyp-Gly-Thi-Ser-D-Tic-Oic-Arg-OH | 6,2 x 10⁻⁹ |
| 19 | Fmoc-(4-aminocyclohexylcarbonyl)-Arg-Pro-Hyp-Gly-Thi-Ser-D-Tic-Oic-Arg-OH | 2,2 x 10⁻⁸ |
| 20 | 7-Theophyllinacetyl-Arg-Pro-Hyp-Gly-Thi-Ser-D-Tic-Oic-Arg-OH | 1,4 x 10⁻⁸ |
| 21 | N-Benzoyl-D-Arg-Arg-Pro-Hyp-Gly-Thi-Ser-D-Tic-Oic-Arg-OH | 8,1 x 10⁻⁹ |
| 22 | Fmoc-Aeg(Fmoc)-Arg-Pro-Hyp-Gly-Thi-Ser-D-Tic-Oic-Arg-OH | 6,6 x 10⁻⁸ |
| 23 | Fmoc-D-Lys-Arg-Pro-Hyp-Gly-Thi-Ser-D-Tic-Oic-Arg-OH | 1,4 x 10⁻⁸ |
| 24 | Fmoc-Arg-Pro-Hyp-Gly-Thi-Ser-D-Tic-Oic-Arg-OH | 2,6 x 10⁻⁸ |
| 25 | Fmoc-Oic-Arg-Pro-Hyp-Gly-Thi-Ser-D-Tic-Oic-Arg-OH | 2,5 x 10⁻⁸ |
| 26 | Fmoc-trans-4-aminomethylcylohexyl-carbonyl-Arg-Pro-Hyp-Gly-Thi-Ser-D-Tic-Oic-Arg-OH | 3,5 x 10⁻⁷ |

Bei einigen ausgewählten Verbindungen wurde in der oben beschriebenen Versuchsanordnung auch die Zeitdauer bestimmt, bis die Gefäßstreifen nach Austausch des peptidhaltigen gegen einen "bloßen" Puffer ihre halbmaximale Empfindlichkeit gegenüber 2 x 10⁻⁷ mol/l Bradykinin wieder erreicht haben. Diese Zeitdauer wird als T₅₀ bezeichnet und ist ein Maßstab für die Wirkdauer. Die rechnerisch ermittelten Werte für diese T₅₀ sind in Tabelle 2 aufgelistet:

**Tabelle 2**

| Bsp. Nr. | Sequenz | T₅₀-Wert (Pulmonar-Arterie) |
|---|---|---|
| 1 | Fmoc-D-Arg-Arg-Pro-Hyp-Gly-Thia-Ser-D-Tic-Oic-Arg-OH | 143,3 min |
| 2 | Fmoc-Aoc-D-Arg-Arg-Pro-Hyp-Gly-Thia-Ser-D-Tic-Oic-Arg-OH | 253,3 min |
| 3 | Dibenzylacetyl-D-Arg-Arg-Pro-Hyp-Gly-Thia-Ser-D-Tic-Oic-Ar g-OH | 67,3 min |
| 4 | Cyclohexylcarbonyl-D-Arg-Arg-Pro-Hyp-Gly-Thia-Ser-D-Tic-Oi c- Arg-OH | 76,7 min |
| 5 | Fmoc-ε-Aminocaproyl-D-Arg-Arg-Pro-Hyp-Gly-Thia-Ser-D-Tic-Oic-Arg-OH | 314,4 min |

Ausgewählte Verbindungen wurden ebenfalls in vivo in dem nachfolgend beschriebenen Test untersucht:

Antiphlogistische Wirkung nach systemischer Gabe: Carrageenan-Pfotenödem an Ratten

### Methode

Als Test für die akute systemische antiphlogistische Wirkung wird das Carrageenan-Pfotenödem an Ratten nach der von Winter C.A. et al., Proc. Soc. Exp. Biol. (N.Y.), 111, 544 (1962) beschriebenen Methode gewählt. Männliche Sprague-Dawley-Ratten im Gewicht um 170 g in Gruppen zu 5 Tieren erhalten die zu prüfenden Substanzen subcutan in destilliertem Wasser gelöst (1 ml/kg Körpergewicht). 15 min später wird nach Messung des Ausgangsvolumens der Pfote in Ether-Narkose in die linke Hinterpfote 0.1 ml 0.5%ige Carrageenan-Lösung injiziert. 3 und 6 Std. später wird die Schwellungszunahme volumetrisch gemessen. Kontrollen erhalten nur das Vehikel. Die Pfotenvolumina sind in ml (Mittelwert und Standardabweichung) angegeben.
Die Ergebnisse sind in Tabelle 3 zusammengefaßt.

**Tabelle 3**

| Bsp. Nr. | Dosis [mg/kg] | Pfotenvolumen | Anstieg des Pfotenvolumens | |
|---|---|---|---|---|
| | | vorher [ml] | nach 3 Std. [ml] | nach 6 Std. [ml] |
| Kontrolle 1 | 0.1 | 1,28 ± 0,06 | 0,67 ± 0,16 | 0,42 ± 0,10 |
| | | 1,35 ± 0,11 | 0,13 ± 0,06 | 0,14 ± 0,05 |
| | 1.0 | 1,36 ± 0,03 | 0,11 ± 0,05 | 0,08 ± 0,01 |
| Kontrolle 2 | 0.1 | 1,33 ± 0,02 | 0,50 ± 0,10 | 0,44 ± 0,03 |
| | | 1,32 ± 0,06 | 0,13 ± 0,04 | 0,29 ± 0,08 |
| | 1.0 | 1,29 ± 0,03 | 0,19 ± 0,06 | 0,27 ± 0,06 |
| Kontrolle 3 | 0.1 | 1,24 ± 0,04 | 0,62 ± 0,11 | 0,54 ± 0,11 |
| | | 1,23 ± 0,05 | 0,19 ± 0,07 | 0,22 ± 0,14 |
| Kontrolle 4 | 0.1 | 1,24 ± 0,04 | 0,62 ± 0,11 | 0,54 ± 0,11 |
| | | 1,21 ± 0,05 | 0,17 ± 0,08 | 0,31 ± 0,10 |
| Kontrolle 5 | 0.1 | 1,33 ± 0,02 | 0,50 ± 0,10 | 0,44 ± 0,03 |
| | | 1,38 ± 0,08 | 0,18 ± 0,04 | 0,20 ± 0,06 |
| | 1.0 | 1,33 ± 0,09 | 0,13 ± 0,03 | 0,17 ± 0,06 |
| Kontrolle 6 | 0.1 | 1,24 ± 0,04 | 0,62 ± 0,11 | 0,54 ± 0,11 |
| | | 1,25 ± 0,02 | 0,31 ± 0,06 | 0,40 ± 0,11 |

Der therapeutische Nutzen der erfindungsgemäßen Peptide umfaßt alle pathologischen Zustände, die durch Bradykinin und Bradykinin verwandte Peptide vermittelt, ausgelöst oder unterstützt werden. Dies beinhaltet u.a. Traumata, wie Wunden, Verbrennungen, Ausschläge, Erytheme, Ödeme, Angina, Arthritis, Asthma, Allergien, Rhinitis, Schock, Entzündungen, Pankreatitis, niedriger Blutdruck, Schmerz, Juckreiz etc. und veränderte Sperma-Motilität.

Die Erfindung betrifft daher auch die Verwendung von Peptiden der Formel I als Heilmittel und pharmazeutische Präparate, die diese Verbindungen enthalten.

Pharmazeutische Präparate enthalten eine wirksame Menge des Wirkstoffs der Formel I - einzeln oder in Kombination - zusammen mit einem anorganischen oder organischen pharmazeutisch verwendbaren Trägerstoff.

Die Anwendung kann enteral, parenteral - wie z. B. subkutan, i. m. oder i. v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Aufgrund der kurzen Halbwertszeiten einiger der beschriebenen Arzneistoffe in Körperflüssigkeiten ist der Einsatz von injizierbaren Retardzubereitungen sinnvoll. Als Arzneiformen können z. B. ölige Kristallsuspensionen, Mikrokapseln, Rods oder Implantate verwendet werden, wobei die letzteren aus gewebeverträglichen Polymeren, insbesondere bioabbaubaren Polymeren, wie z. B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,001-5 mg/kg, bei systemischen Applikationsformen sind 0,001-10 mg/kg geeignet.

### Verzeichnis der Abkürzungen:

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Acm: Acetamidomethyl
- Aeg: N-(2-Aminoethyl)glycin
- ε-Ahx: ε-Aminohexanoyl
- Aoc: cis, endo-2-Azabicyclo[3.3.0]octan-3-S-carbonyl
- Boc: tert.-Butyloxycarbonyl
- But: tert.-Butyl
- Bzl: Benzyl
- Cbz: Benzyloxycarbonyl
- CDF: Chlor-(D)-phenylalanyl
- Cha: Cyclohexylalanyl
- Chg: Cyclohexylglycyl
- Cl-Z: 4-Chlor-benzyloxycarbonyl
- Dic: Dihydroindolcarbonyl
- DMF: Dimethylformamid
- DOMT: O-Methyl-(D)-Threonyl
- Dnp: 2,4-Dinitrophenyl
- Fmoc: 9-Fluorenylmethyloxycarbonyl
- MDY: O-Methyl-(D)-Tyrosyl
- Me: Methyl
- 4-Mebzl: 4-Methylbenzyl
- Mtr: 4-Methoxy-2,3,6-trimethylphenyl-sulfonyl
- Mts: Mesitylen-2-sulfonyl
- Nal: 2-Naphthylalanyl
- NMP: N-Methylpyrrolidin
- Npg: Neopentylglycyl
- Oic: cis-endo-Octahydroindol-2-carbonyl
- Opr: Isoxazolidin-3-ylcarbonyl
- Pal: Pyridylalanyl
- Pmc: 2,2,5,7,8-Pentamethylchroman-6-sulfonyl
- Tbg: tert.-Butylglycyl
- TBTU: 2-(1H-Benzotriazol-1-yl)-1,1,3,3-tetramethyluroniumtetrafluorborat
- tBu: tert.-Butyl
- Tcs: 4-Methylphenylsulfonyl
- TFA: Trifluoressigsäure
- Thia: 2-Thienylalanyl
- Tic: 1,2,3,4-Tetrahydroisochinolin-3-ylcarbonyl
- TOTU: O-[(Cyano-(ethoxycarbonyl)methyliden)amino]-1,1,3,3,-tetramethyluroniumtetrafluorborat
- Trt: Trityl

Die folgenden Beispiele sollen die bevorzugten Methoden nach Festphasen-Synthese der erfindungsgemäßen Peptide verdeutlichen, ohne daß die Erfindung darauf eingeschränkt wäre.

### Man verwendete unter anderem folgende Aminosäure-Derivate:

Fmoc-Arg(Mtr)-OH, Fmoc-D-Arg(Mtr)-OH, Fmoc-D-Arg(Pmc)-OH, Boc-(D)-Arg-OH, Fmoc-Arg(Pmc)-OH, Fmoc-Hyp-OH, Fmoc-Pro-OObt, Fmoc-Gly-OObt, Fmoc-Phe-OObt, Fmoc-Ser(tBu)-OObt, Fmoc-(D)-Tic-OH, Fmoc-Gln-OH, Fmoc-Aoc-OH, Fmoc-Thia-OH, Fmoc-Oic-OH, Fmoc-Leu-OH, Fmoc-Lys(Boc)-OH, Fmoc-ε-Ahx-OH.

### Beispiel 1:

### Fmoc-D-Arg-Arg-Pro-Hyp-Gly-Thia-Ser-D-Tic-Oic-Arg-OH

Wurde mit einem Peptid-Synthesizer Modell 430 A der Fa. Applied Biosystems unter Verwendung der Fmoc-Methode an einem mit Fmoc-Arg(Mtr)-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 mol/l Lösung von Diisopropylcarbodiimid in DMF.
Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Als letztes Aminosäurederivat wurde Fmoc-D-Arg(Pmc)-OH aufgekuppelt und anschließend nicht mit Piperidin entschützt.
Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert. Der verbliebene Rückstand wurde an ®Sephadex LH 20 mit 10 %iger Essigsäure chromatographiert. Die das reine Peptid enthaltenden Fraktionen wurden vereinigt und gefriergetrocknet
MS(FAB) : 1526.9

### Beispiel 2:

Fmoc-Aoc-D-Arg-Arg-Pro-Hyp-Gly-Thia-Ser-D-Tic-Oic-Arg-OH

Die Titelverbindung wurde dadurch erhalten, daß man von einem vorher analog zu Beispiel 1 erhaltenem Peptid-Harz mit der Sequenz Fmoc-D-Arg(Pmc)-Arg(Mtr)-Pro-Hyp-Gly-Thia-Ser(But)-D-Tic-Oic-Arg(Mtr)-Harz zunächst die N-terminale Fmoc-Schutzgruppe mittels Piperidin/Dimethylformamid abspaltete, das Harz mit DMF wusch und dann Fmoc-Aoc-OH unter Verwendung des Uronium-Kupplungsreagenzes TOTU aufkuppelte. Die Abspaltung des Peptides vom Harz erfolgte dann mit einem Gemisch aus Trifluoressigsäure, Trimethylsilylbromid und m-Kresol. Die Reinigung erfolgte analog zu dem in Beispiel 1 beschriebenen Verfahren.
MS(FAB) : 1663,8

Die folgenden Beispiele wurden analog Beispiel 2 hergestellt

### Beispiel 3:

Dibenzylacetyl-D-Arg-Arg-Pro-Hyp-Gly-Thia-Ser-D-Tic-Oic-Arg-OH
MS(FAB): 1526,7

### Beispiel 4:

Cyclohexylcarbonyl-D-Arg-Arg-Pro-Hyp-Gly-Thia-Ser-D-Tic-Oic-Arg-OH
MS(FAB): 1414,7

### Beispiel 5:

Fmoc-ε-Aminohexanoyl-D-Arg-Arg-Pro-Hyp-Gly-Thia-Ser-D-Tic-Oic-Arg-OH
MS(FAB): 1639,6

### Beispiel 6:

N,N-Dibenzyl-Glycyl-D-Arg-Arg-Pro-Hyp-Gly-Thia-Ser-D-Tic-Oic-Arg-OH
MS(FAB): 1542,2

### Beispiel 7:

Fmoc-D-Aoc-Arg-Arg-Pro-Hyp-Gly-Thi-Ser-D-Tic-Oic-Arg-OH
MS(FAB): 1663,8

### Beispiel 8:

2-(4-lsobutylphenyl)-propionyl-Arg-Arg-Pro-Hyp-Gly-Thi-Ser-D-Tic-Oic-Arg-OH
MS(FAB): 1493

### Beispiel 9:

(2-R-(tert.-butylsulfonylmethyl)-3-(1-naphthyl)propionyl-Arg-Arg-Pro-Hyp-Gly-Thi-Ser-D-Tic-Oic-Arg-OH
MS(FAB): 1621

### Beispiel 10:

Indol-3-yl-acetyl-Arg-Arg-Pro-Hyp-Gly-Thi-Ser-D-Tic-Oic-Arg-OH
FS(MAB): 1462

### Beispiel 11:

2-(4-lsobutylphenyl)propionyl-D-Arg-Arg-Pro-Hyp-Gly-Thi-Ser-D-Tic-Oic-Arg-OH
FS(MAB): 1493

### Beispiel 12:

2-(4-lsobutylphenyl)propionyl-6-aminohexanoyl-D-Arg-Arg-Pro-Hyp-Gly-Thi-Ser-D- Tic-Oic-Arg-OH
FS(MAB): 1606

### Beispiel 13:

6-(4-Benzoyl-benzoylamino)hexanoyl-D-Arg-Arg-Pro-Hyp-Gly-Thi-Ser-D-Tic-Oic-Arg-OH
FS(MAB): 1625,8

### Beispiel 14:

Fmoc-Aeg(Fmoc)-D-Arg-Arg-Pro-Hyp-Gly-Thi-Ser-D-Tic-Oic-Arg-OH
FS(MAB): 1849,8

### Beispiel 15:

Fmoc-(4-aminocyclohexylcarbonyl)-D-Arg-Arg-Pro-Hyp-Gly-Thi-Ser-D-Tic-Oic-Arg-OH
FS(MAB): 1652

### Beispiel 16:

1,8-Naphthalimidoacetyl-D-Arg-Arg-Pro-Hyp-Gly-Thi-Ser-D-Tic-Oic-Arg-OH
FS(MAB): 1542,3

### Beispiel 17:

(2-R-(tert.-butylsulfonylmethyl)-3-(1-naphthyl)propionyl-D-Arg-Arg-Pro-Hyp-Gly-Thi-Ser-D-Tic-Oic-Arg-OH
FS(MAB): 1621,9

### Beispiel 18:

Indol-3-yl-acetyl-D-Arg-Arg-Pro-Hyp-Gly-Thi-Ser-D-Tic-Oic-Arg-OH
FS(MAB): 1462

### Beispiel 19:

Fmoc-(4-aminocyclohexylcarbonyl)-Arg-Pro-Hyp-Gly-Thi-Ser-D-Tic-Oic-Arg-OH
FS(MAB): 1496

### Beispiel 20:

7-Theophyllinacetyl-Arg-Pro-Hyp-Gly-Thi-Ser-D-Tic-Oic-Arg-OH
FS(MAB): 1369

### Beispiel 21:

N-Benzoyl-D-Arg-Arg-Pro-Hyp-Gly-Thi-Ser-D-Tic-Oic-Arg-OH
FS(MAB): 1410

### Beispiel 22:

Fmoc-Aeg(Fmoc)-Arg-Pro-Hyp-Gly-Thi-Ser-D-Tic-Oic-Arg-OH
FS(MAB): 1694

### Beispiel 23:

Fmoc-D-Lys-Arg-Pro-Hyp-Gly-Thi-Ser-D-Tic-Oic-Arg-OH
FS(MAB): 1500

### Beispiel 24:

Fmoc-Arg-Pro-Hyp-Gly-Thi-Ser-D-Tic-Oic-Arg-OH
FS(MAB): 1371

### Beispiel 25:

Fmoc-Oic-Arg-Pro-Hyp-Gly-Thi-Ser-D-Tic-Oic-Arg-OH
FS(MAB): 1523

### Beispiel 26:

Fmoc-trans-4-aminomethylcyclohexyl-carbonyl-Arg-Pro-Hyp-Gly-Thi-Ser-D-Tic-Oic-Arg-OH
FS(MAB): 1511

## Patentansprüche

1. Peptid der Formel I
Z-P-A-B-C-E-F-K-(D)Q-G-M-F'-I (I),
in welcher
Z Fmoc, Dibenzylacetyl, Cyclohexylcarbonyl, N,N-Dibenzyl-glycyl, 2-(4-Isobutylphenyl)propionyl, (2-R-(tert.-Butylsulfonylmethyl)-3-(1-naphthyl)-propionyl, Indol-3-yl-acetyl, (4-Benzoyl-benzoyl), 1,8-Naphthalimidoacetyl, 7-Theophyllinacetyl oder N-Benzoyl bedeutet;
P für eine direkte Bindung, Aoc, ε-Aminohexanoyl, D-Aoc, Aeg(Fmoc), 4-Aminocyclohexylcarbonyl, 4-Aminomethylcyclohexylcarbonyl oder Oic steht;
A (D)- oder (L)-Arg, (D)- oder (L)-Lys oder eine Bindung bedeutet;
B Arg bedeutet;
C Pro-Hyp-Gly bedeutet;
E Thia bedeutet;
F Ser bedeutet;
K für eine direkte Bindung steht;
Q Tic bedeutet;
M für eine direkte Bindung steht;
G für cis-endo-, cis-exo-, trans-Octahydroindol-2-carbonsäure steht;
F' Arg bedeutet und
I für OH steht;
sowie deren physiologisch verträgliche Salze.

2. Verfahren zur Herstellung eines Peptids der Formel I gemäß Anspruch 1, dadurch gekennzeichnet, daß man
a) ein Fragment mit C-terminaler freier Carboxylgruppe oder dessen aktiviertes Derivat mit einem entsprechenden Fragment mit N-terminaler freier Aminogruppe umsetzt oder
b) das Peptid stufenweise aufbaut,
in der nach (a) oder (b) erhaltenen Verbindung gegebenenfalls eine oder mehrere zum Schutz anderer Funktionen temporär eingeführte Schutzgruppen abspaltet und die so erhaltenen Verbindungen der Formel I gegebenenfalls in ihr physiologisch verträgliches Salz überführt.

3. Verwendung eines Peptids der Formel I gemäß Anspruch 1 zur Herstellung eines Heilmittels.

4. Verwendung eines Peptids der Formel I gemäß Anspruch 1 zur Herstellung eines Heilmittels zur Behandlung von pathologischen Zuständen, die durch Bradykinin und Bradykinin verwandte Peptide vermittelt, ausgelöst oder unterstützt werden.

5. Pharmazeutisches Mittel enthaltend ein Peptid der Formel I gemäß Anspruch 1.

## Claims

1. A peptide of the formula I
Z-P-A-B-C-E-F-K-(D)Q-G-M-F'-I (I),
in which
Z is Fmoc, dibenzylacetyl, cyclohexylcarbonyl, N,N-dibenzyl-glycyl, 2-(4-isobutylphenyl)-propionyl, (2-R-(tert-butylsulfonylmethyl)-3-(1-naphthyl)propionyl, indol-3-yl-acetyl, (4-benzoyl-benzoyl), 1,8-naphthalimidoacetyl, 7-theophyllineacetyl or N-benzoyl;
P is a direct linkage, Aoc, ε-aminohexanoyl, D-Aoc, Aeg(Fmoc), 4-aminocyclohexylcarbonyl, 4-aminomethylcyclohexylcarbonyl or Oic;
A is (D)- or (L)-Arg, (D)- or (L)-Lys or a bond;
B is Arg;
C is Pro-Hyp-Gly;
E is Thia;
F is Ser;
K is a direct linkage;
Q is Tic;
M is a direct linkage;
G is cis-endo-, cis-exo-, trans-octahydroindole-2-carboxylic acid;
F' is Arg and
I is OH;
and the physiologically tolerated salts thereof.

2. A process for preparing a peptide of the formula I as claimed in claim 1, which comprises
a) reacting a fragment with a C-terminal free carboxyl group or its activated derivative with a corresponding fragment with an N-terminal free amino group or
b) assembling the peptide stepwise,
where appropriate eliminating in the compound obtained as in (a) or (b) one or more protective groups introduced temporarily to protect other functionalities, and where appropriate converting the compounds of the formula I obtained in this way into the physiologically tolerated salt thereof.

3. The use of a peptide of the formula I as claimed in claim 1 for the preparation of a medicine.

4. The use of a peptide of the formula I as claimed in claim 1 for the preparation of a medicine for the treatment of pathological states which are mediated, induced or assisted by bradykinin and peptides related to bradykinin.

5. A pharmaceutical composition containing a peptide of the formula I as claimed in claim 1.

## Revendications

1. Peptide de formule I
Z-P-A-B-C-E-F-K-(D)Q-G-M-F'-I (I)
dans laquelle
Z représente le groupe Fmoc, dibenzylacétyle, cyclohexylcarbonyle, N,N-dibenzylglycyle, 2-(4-isobutylphényl)-propionyle, (2-R-(tert-butylsulfonylméthyl)-3-(1-naphtyl)propionyle, indol-3-yl-acétyle, (4-benzoylbenzoyle), 1,8-naphtalimidoacétyle, 7-théophyllinacétyle ou N-benzoyle;
P représente une liaison directe ou le groupe Aoc, ε-aminohexanoyle, D-Aoc, Aeg(Fmoc), 4-aminocyclohexylcarbonyle, 4-aminométhylcyclohexylcarbonyle ou Oic;
A représente (D)- ou (L)-Arg, (D)- ou (L)-Lys ou une liaison;
B représente Arg;
C représente Pro-Hyp-Gly;
E représente Thia;
F représente Ser;
K représente une liaison directe;
Q représente Tic;
M représente une liaison directe;
G représente l'acide *cis*-endo-, *cis*-exo-, *trans*-octahydro-indole-2-carboxylique;
F' représente Arg et
I représente OH;
ainsi que ses sels physiologiquement acceptables.

2. Procédé pour la préparation d'un peptide de formule I selon la revendication 1, caractérisé en ce que
a) on fait réagir un fragment à groupe carboxyle C-terminal libre, ou un dérivé activé de celui-ci, avec un fragment correspondant à groupe amino N-terminal libre, ou
b) on fait graduellement la synthèse du peptide,
dans le composé obtenu selon (a) ou (b), éventuellement on élimine un ou plusieurs groupes protecteurs temporairement introduits pour la protection d'autres fonctions, et éventuellement on convertit les composés de formule I ainsi obtenus en un de leurs sels physiologiquement acceptables.

3. Utilisation d'un peptide de formule I selon la revendication 1, pour la fabrication d'un médicament.

4. Utilisation d'un peptide de formule I selon la revendication 1, pour la fabrication d'un médicament destiné au traitement d'états pathologiques qui sont médiés, déclénchés ou favorisés par la bradykinine et des peptides apparentés à la bradykinine.

5. Produit pharmaceutique contenant un peptide de formule I selon la revendication 1.
